**Europäisches Patentamt**

**European Patent Office**

**Office européen des brevets**

(11) Veröffentlichungsnummer: **0 075 062**
**A1**

---

## EUROPÄISCHE PATENTANMELDUNG

(21) Anmeldenummer: **81890152.2**

(22) Anmeldetag: **18.09.81**

(51) Int. Cl.³: **A 61 M 31/00, A 61 M 5/14**

---

(43) Veröffentlichungstag der Anmeldung: **30.03.83**
**Patentblatt 83/13**

(84) Benannte Vertragsstaaten: **CH DE FR GB IT LI SE**

(71) Anmelder: **Sadjak, Anton Dr., Leonhardstrasse 100 a/24, A-8010 Graz (AT)**

(72) Erfinder: **Sadjak, Anton Dr., Leonhardstrasse 100 a/24, A-8010 Graz (AT)**

---

(54) **In den Körper implantierbare Einrichtung zur konstanten Abgabe eines Wirkstoffs.**

(57) Bei einer in den Körper implantierbaren Einrichtung zur konstanten Abgabe eines Wirkstoffs ist die Kammer (1), die das Wirkstoffreservoir darstellt, durch eine bewegliche, undurchlässige Membran (4) von der mit einer Treibsubstanz gefüllten Kammer (2) getrennt. Weiters ist die Kammer (1) mit einer verschließbaren Nachfüllöffnung (3) und einer Ausströmöffnungseinrichtung (6) versehen.

Der erforderliche Druck, der durch den Übergang von der flüssigen zur gasförmigen Phase der Treibsubstanz zur Abgabe eines Wirkstoffs bereitgestellt wird, ist temperaturabhängig. Durch die ziemlich gleichbleibende Körpertemperatur kann er als konstant angesehen werden. Durch eine transcutane Nachfüllung des bereits abgegebenen Wirkstoffs wird gleichzeitig die Treibsubstanz in der Kammer (2) von der gasförmigen Phase in die flüssige Phase zurückgeführt.

Das Gehäuse (5) der implantierbaren Einrichtung wird mit einem gut verträglichen Material (7) überzogen bzw. gefertigt.

- 1 -

**In den Körper implantierbare Einrichtung zur konstanten Abgabe eines Wirkstoffs.**

Die übliche Applikation von Medikamenten in Form von Tabletten, Pillen, Salben, Tropfen und Injektionen zur Chemotherapie von Krankheiten ist schon sehr alt. Pillen kannten schon die alten Ägypter, über Salben und Einreibungen gibt es Informationen aus der Zeit 2ooo v.Christi. Zäpfchen kennt man seit Jahrhunderten und die Inhalation von Wirkstoffen wurde im Mittelalter praktiziert. Die Injektionstechnik gibt es seit dem 17. Jahrhundert.

Die herkömmlichen Applikationsformen haben den Nachteil der anfänglich sehr schnellen Abgabe des Wirkstoffs in den Organismus und die darauf ständig abnehmende Arzneimittelkonzentration. Dieser Wechsel von Über- und Unterdosierung hat einen schwankenden Serumspiegel des applizierten Wirkstoffs zur Folge, der in Verbindung mit unerwünschten Nebeneffekten steht. Durch die kurze Abgabedauer und Halbwertszeit (Stundenbereich) herkömmlicher Applikationsverfahren muß die Medikamentengabe häufig wiederholt werden. Dadurch wird oft die optimale therapeutische Wirkung nicht erreicht, oder nur auf Kosten toxischer Nebenwirkungen.

Eigene tierexperimentelle Untersuchungen zeigten im Vergleich zur üblichen Injektionstechnik eine wesentlich höhere Wirksamkeit einer permanent über längere Zeit mit einer "Diffusionskapsel" applizierten Substanz. Der Wirkstoff tritt in gelöster Form aus und wird in den inneren Kreislauf abgegeben. Die unterschiedliche gastrointestinale Absorption bei herkömmlicher Medikamentengabe wird so verhindert. Weiters wird bei so einer Wirkstoffgabe – direkt in das System – die vorherige Metabolisierung durch die Leber vermieden. Die Dosierbarkeit der permanenten Wirkstoffgabe (systemisch oder lokal appliziert) ermöglicht eine Konzentration im Organismus, die unterhalb des toxischen Bereiches liegt.

Die pharmazeutischen Unternehmen liefern eine Vielzahl starker und stärkster Medikamente und ihre Zahl steigt ständig. Statistiken zeigen, daß ein hoher Prozentsatz von Patienten regelmäßig Überdosen schluckt. Doch selbst wenn der Kranke die Vorschrift des Arztes befolgt, nimmt er mehr chemische Substanzen zu sich als zur Heilung erforderlich wäre. Die Ursache dieser Diskrepanz liegt in der Applikationsform.

Zur Behandlung der meisten Krankheiten sind die üblichen Applikationsmethoden ausreichend. In vielen Fällen jedoch sind diese Methoden, mit denen man keinen gleichmäßigen Plasmaspiegel der Wirksubstanz längere Zeit aufrechterhalten kann, für eine ideale Behandlung nicht geeignet. Es gibt einige Krankheiten, die eine gleichmässige, länger anhaltende Abgabe der Wirksubstanz erfordern.

Dieses Abgabesystem beschränkt sich nicht auf die Chemotherapie von Krankheiten allein – die Anwendungsgebiete sind sehr umfangreich. Eine permanente, konstante Applikation von Wirkstoffen eröffnet neue Möglichkeiten besonders zur Bearbeitung pharmakologischer bzw. pathophysiologischer Fragestellungen, z.B. für die Charakterisierung spontaner oder artifiziell provozierter Schäden und deren pharmakologische Behandlung im Tierversuch. Anwendungsmöglichkeiten ergeben sich in der Pharmakologie, Toxikologie, Physiologie, Endokrinologie, Immunologie und zur Prüfung der Wirkung neuer Pharmaka.

Die gleichmäßige Applikation vieler Medikamente ist wesentlich wirksamer als eine ein- oder mehrmalige Einnahme nach der "üblichen" Arzneimittelgabe. Viele Medikamente können nur intravenös verabreicht werden, da bei oraler Aufnahme deren Wirksamkeit bei Passage des Magen-Darmtraktes abgeschwächt wird bzw. verloren geht. Momentan ist die intravenöse Infusion die gängigste Form einer gleichmäßigen Medikamentengabe. Zur Zeit wird in vielen Labors an der Entwicklung neuer Depotsysteme gearbeitet. Depotformen wurden entwickelt, bei denen die langsame Abgabe der Wirksubstanz durch Diffusion aus Kunststoffkapseln und Gelen erzielt wird. Auch Wirkstofftabletten, bestehend aus reinem Wirkstoff, der sich langsam auflöst, stellt eine weitere Speicherform dar, ist aber abhängig von der Löslichkeit des Medikamentes in der Körperflüssigkeit und daher nur für bestimmte Wirkstoffe anwendbar. Osmotisch und elektroosmotisch wirkende Depotformen werden angewandt, eine konstante Dosierung über lange Zeit ist auf Grund der irreversiblen Erschöpfbarkeit dieser Systeme jedoch nicht möglich. Das Problem, die Energie für langarbeitende Systeme zu erzeugen bzw. zu konservieren, ist größtenteils ungelöst.

In den AT-Psen 332563 und 334543 werden Wirkstoffspender beschrieben, die ausschließlich nach dem Osmose-Prinzip arbeiten. Die Wand dieser Spender, die ganz oder zum Teil semipermeabel und dadurch durchlässig für die vorhandene Außenflüssigkeit (Körperflüssigkeit) ist, enthält eine osmotisch wirksame Substanz. Der auf Grund eines Osmosevorganges entstehende osmotische Druck wirkt auf die undurchlässige Wand des Wirkstoff-Reservoirs und bewirkt dadurch eine kontinuierliche Abgabe eines Wirkstoffes. Naturgemäß stellt sich nach einer bestimmten Zeit ein Äquilibrium der osmotisch wirksamen Substanzen ein. Damit ist dieses System irreversibel erschöpft! Dieses erschöpfbare, nicht regenerierbare System ist für viele Anwendungsgebiete deshalb nicht bzw. nur zum Teil brauchbar und setzt dadurch deren Anwendung enge Grenzen.

Nach der vorliegenden Erfindung wird eine Einrichtung geschaffen, mit der eine gezielte, konstante Abgabe eines Wirkstoffes über eine beliebig lange Zeit erzielt werden kann. Die Erfindung betrifft eine in den Körper implantierbare Einrichtung zur konstanten Abgabe eines Wirkstoffes, bestehend aus zwei in einem gewebefreundlichen Gehäuse angeordneten und durch eine Membran getrennte Kammern, wobei die eine Kammer den Wirkstoff enthält und mit einer Ausströmöffnungseinrichtung zur Abgabe dieses Wirkstoffs versehen ist, welche Einrichtung dadurch gekennzeichnet ist, daß die eine, den Wirkstoff enthaltende Kammer mit einer verschließbaren Nachfüllöffnung versehen ist und daß die andere, wirkstofffreie Kammer zur Ausübung eines Druckes auf die Membran und die dadurch hervorrufbare Abgabe des Wirkstoffes aus der Kammer durch die Austrittsöffnungseinrichtung eine isobar expandierbare Treibsubstanz enthält. Die Erfindung schafft somit einen beliebig lang arbeitenden Wirkstoffspender.

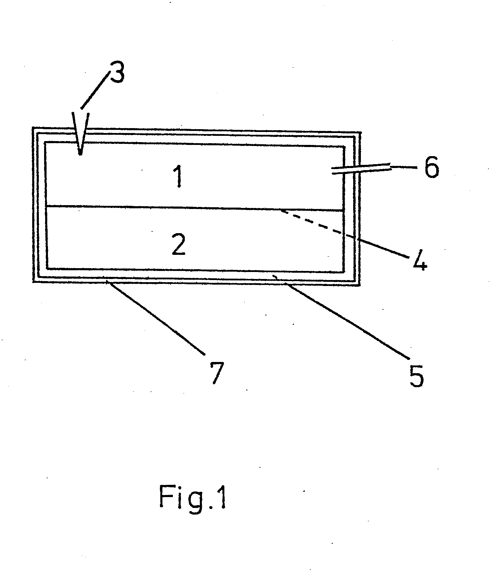

Die Zeichnungsfigur (Fig. 1) zeigt die schematische Darstellung der erfindungsgemäßen Vorrichtung. Sie besteht aus der Kammer (1) für die Wirkstoffmenge, die weiters mit einer verschließbaren Nachfüllöffnung (3) und zur Abgabe eines Wirkstoffes mit der Ausströmöffnungseinrichtung (6) versehen ist. Die Kammer (2) für die Treibsubstanz ist durch eine bewegliche, undurchlässige Membran (4) von der Wirkstoffkammer (1) getrennt. Das Gehäuse (5) der Vorrichtung wird mit einem gut verträglichen, gewebefreundlichen Material (7) überzogen.

Bei dessen Anwendung wird mit dem Abfüllen der abzugebenden Wirkstoffmenge in die Kammer (1) ein Druck in der Kammer (2) erzeugt, der die Treibsubstanz von der gasförmigen Phase in die flüssige Phase zurückführt. Der zur Abgabe der Wirkstoffmenge erforderliche konstante Druck wird durch den Übergang von der flüssigen zur gasförmigen Phase der Treibsubstanz bereitgestellt. Eine Flüssigkeit, die mit ihrem Dampf im Gleichgewicht steht, übt einen gleichbleibenden Druck aus, der nur temperaturabhängig ist. Da die Körpertemperatur stets ziemlich gleich ist, so trifft dies auch für den Druck in der Pumpe zu. Auf diesem Wege wird nach Implantation der Wirkstoffe (Medikament) dosiert beliebig lange an eine bestimmte Stelle des Körpers oder in ein Organ abgegeben. Durch transcutane Nachfüllung kann jederzeit die Konzentration des Wirkstoffes geändert werden.

0075062

- 1 -

Patentansprüche:

1. In den Körper implantierbare Einrichtung zur konstanten Abgabe eines Wirkstoffs, bestehend aus zwei in einem gewebefreundlichen Gehäuse angeordneten und durch eine Membran getrennte Kammern, wobei die eine Kammer den Wirkstoff enthält und mit einer Ausströmöffnungseinrichtung zur Abgabe dieses Wirkstoffs versehen ist,
dadurch gekennzeichnet,
daß die eine, den Wirkstoff enthaltende Kammer (1) mit einer verschließbaren Nachfüllöffnung (3) versehen ist und daß die andere, wirkstoffreie Kammer (2) zur Ausübung eines Druckes auf die Membran (4) und die dadurch hervorrufbare Abgabe des Wirkstoffs aus der Kammer (1) durch die Ausströmöffnungseinrichtung (6) eine isobar expandierbare Treibsubstanz enthält.

2. Vorrichtung nach Patentanspruch 1,
dadurch gekennzeichnet,
daß als verschließbare Nachfüllöffnung (3) ein Ventil vorgesehen ist.

BAD ORIGINAL

Fig.1

# EUROPÄISCHER RECHERCHENBERICHT

0075062

Nummer der Anmeldung

EP 81 89 0152

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | US - A - 3 840 009 (MICHAELS et al.)<br><br>* Abbildungen 1-3; Spalte 1, Zeilen 6-14; Spalte 2, Zeilen 43-56, 62-66; Spalte 3, Zeile 66 bis Spalte 4, Zeile 2; Spalte 4, Zeilen 14-28, 50-53, 60-63; Spalte 6, Zeilen 3-42, 58-62; Spalte 12, Zeilen 8 und 9 *<br>--- | 1,2 |
| X | US - A - 2 876 768 (H.C. SCHULTZ)<br><br>* Abbildungen 1,2,4; Spalte 1, Zeilen 15-42; Spalte 4, Zeilen 25-28; Spalte 4, Zeile 75 bis Spalte 5, Zeile 3; Spalte 6, Zeile 37 bis Spalte 7, Zeile 2; Spalte 7, Zeilen 42-47 *<br>--- | 1,2 |
| X | US - A - 3 786 813 (A.S. MICHAELS)<br><br>* Abbildungen 1-6; Spalte 3, Zeilen 31-45; Spalte 6, Zeilen 39-45; Spalte 9, Zeilen 8-14 *<br>--- | 1 |
| X | US - A - 3 788 322 (ALAN S. MICHAELS)<br><br>* Abbildungen 1-7; Spalte 1, Zeilen 9-37; Spalte 5, Zeilen 16-20; Spalte 9, Zeile 62 bis Spalte 10, Zeile 17 *<br>--- | 1 |
| | US - A - 3 797 492 (V.A. PLACE)<br><br>* Abbildung 4; Spalte 1, Zeilen 21-37; Spalte 8, Zeilen 12-35; Spalte 9, Zeilen 55-62 * | 1 |

Der vorliegende Recherchenbericht wurde für alle Patentansprüche erstellt.

| Recherchenort | Abschlußdatum der Recherche | Prüfer |
|---|---|---|
| Den Haag | 07.01.1982 | DURAND-SMET |

**KLASSIFIKATION DER ANMELDUNG (Int. Cl.³)**

A 61 M 31/00
A 61 M 5/14

**RECHERCHIERTE SACHGEBIETE (Int. Cl.³)**

A 61 M

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

EPA form 1503.1   06.78